# EUROPEAN PATENT SPECIFICATION

(11) **EP 4 389 603 B1**
(45) Date of publication and mention of the grant of the patent: **09.09.2026**
(21) Application number: 23218196.6
(22) Date of filing: 19.12.2023
(51) Int. Cl.: B64D 13/06

(54) **IMPROVED AIR MANAGEMENT SYSTEM WITH NEGATIVE ION TECHNOLOGY FOR IMPROVED AIR QUALITY**
VERBESSERTES LUFTVERWALTUNGSSYSTEM MIT NEGATIVER IONENTECHNOLOGIE FÜR VERBESSERTE LUFTQUALITÄT
SYSTÈME DE GESTION D'AIR AMÉLIORÉ AVEC TECHNOLOGIE D'IONS NÉGATIFS POUR UNE QUALITÉ D'AIR AMÉLIORÉE

(30) Priority: 21.12.2022 IN 202211074226
(43) Date of publication of application: 26.06.2024
(73) Proprietor: Hamilton Sundstrand Corporation, Charlotte, NC 28217-4578 (US)
(72) Inventor: KOMARAGIRI, Venkatesh, 560037 Bangalore, Karnataka (IN); REDDY, Sangati Surendra, 522003 Guntur (IN); MOHANTY, Indramani, 759100 Talcher, Odisha (IN)
(74) Representative: Dehns

(56) References cited:
- US-A1- 2003 150 222
- US-A1- 2004 007 000

## Description

This application claims the benefit of IN Application No. 202211074226 filed December 21, 2022.

### TECHNICAL FIELD

The present disclosure relates to air management systems, assemblies and methods and, more particularly, to air management systems, assemblies and methods for aircraft, with the air management systems/assemblies having negative ion technology for improved air quality.

### BACKGROUND

In general, the cabin air supply on most large commercial aircraft is provided using engine bleed air systems. There is evidence that some people experience acute symptoms due to a fume event or exposure to contaminants in aircraft cabin air.

Currently available air management systems typically cannot handle the toxic chemicals/gases and/or harmful viruses/bacteria, and a significant percentage of the viruses will pass through the filters (e.g., high-efficiency particulate air or HEPA filters). As such, this may cause the outbreak of harmful viruses/bacteria (e.g., SARS-CoV-2 or the like). US 2004/0007000 A1 describes an air refining device and ion generator used for the device. US 2003/0150222 A1 describes an air treatment system for airplanes.

### BRIEF DESCRIPTION

The present disclosure provides for air management systems, assemblies and methods. More particularly, the present disclosure provides for aircraft air management systems and methods, with the air management systems/assemblies having negative ion technology for improved air quality.

The present invention provides in a first aspect for an air management system including a mixer unit in fluid communication with an aircraft cabin, and the mixer unit in fluid communication with a negative ion generator and with filtered air from the aircraft cabin; wherein the negative ion generator is configured to generate negative ions that mix with the filtered air from the aircraft cabin in the mixer unit, and the mixer unit is configured to send the mixed air to the aircraft cabin; and wherein after joint union or absorption of the generated negative ions with positive ions from the mixed air, the joint union or absorption of the generated negative ions with the positive ions inhibits growth of bacterial or virus activity and reduces concentrations of toxic gases or chemicals in the mixed air in the aircraft cabin, and wherein the negative ion generator is configured to generate negative hydroxyl radicals or ions and nano-sized capsules of water enveloping amounts of the negative hydroxyl radicals or ions.

In a preferred embodiment, the joint union or absorption of the generated negative ions with the positive ions inhibits growth of SARS-CoV-2 in the mixed air in the aircraft cabin.

In a preferred embodiment, the joint union or absorption of the generated negative ions with the positive ions reduces concentrations of at least one of smoke, vapor, haze, fumes, dust, allergens, abnormal odors, carbon monoxide, methane or tricresyl phosphate in the mixed air in the aircraft cabin.

In a preferred embodiment, the joint union or absorption of the generated negative ions with the positive ions inhibits growth of microbes or airborne bacteria.

In a preferred embodiment, the negative hydroxyl radicals or ions react by transforming hydrogen from components in the mixed air into water.

In a preferred embodiment, the mixer unit is in fluid communication with a cooled air stream from a turbine of an air cycle machine.

In a preferred embodiment, the air cycle machine comprises a compressor, and the compressor is in fluid communication with an air feed stream; and the air feed stream comprises a bleed air feed stream from a bleed air system or comprises an ambient air feed stream from a no-bleed system.

In a preferred embodiment, the turbine is in fluid communication with a ram air stream that is fed to the turbine via at least one main heat exchanger.

The present invention provides in a second aspect for a method for operating an air management system including providing a mixer unit in fluid communication with an aircraft cabin, and the mixer unit in fluid communication with a negative ion generator and with filtered air from the aircraft cabin; and generating negative ions via the negative ion generator; and mixing the generated negative ions with the filtered air from the aircraft cabin in the mixer unit; and sending the mixed air to the aircraft cabin via the mixer unit; and wherein after joint union or absorption of the generated negative ions with positive ions from the mixed air, the joint union or absorption of the generated negative ions with the positive ions inhibits growth of bacterial or virus activity and reduces concentrations of toxic gases or chemicals in the mixed air in the aircraft cabin, and the negative ion generator generates negative hydroxyl radicals or ions and nano-sized capsules of water enveloping amounts of the negative hydroxyl radicals or ions.

In a preferred embodiment, the joint union or absorption of the generated negative ions with the positive ions inhibits growth of SARS-CoV-2 in the mixed air in the aircraft cabin.

In a preferred embodiment, the joint union or absorption of the generated negative ions with the positive ions reduces concentrations of at least one of smoke, vapor, haze, fumes, dust, allergens, abnormal odors, carbon monoxide, methane or tricresyl phosphate in the mixed air in the aircraft cabin.

In a preferred embodiment, the joint union or absorption of the generated negative ions with the positive ions inhibits growth of microbes or airborne bacteria.

In a preferred embodiment, the negative hydroxyl radicals or ions react by transforming hydrogen from components in the mixed air into water.

In a preferred embodiment, the mixer unit is in fluid communication with a cooled air stream from a turbine of an air cycle machine and the air cycle machine comprises a compressor, and the compressor is in fluid communication with an air feed stream; and the air feed stream comprises a bleed air feed stream from a bleed air system or comprises an ambient air feed stream from a no-bleed system, or the turbine is in fluid communication with a ram air stream that is fed to the turbine via at least one main heat exchanger.

The above described and other features are exemplified by the following figures and detailed description.

Additional features, functions and applications of the disclosed systems, assemblies and methods of the present disclosure will be apparent from the description which follows, particularly when read in conjunction with the appended figures.

### BRIEF DESCRIPTION OF THE DRAWINGS

The following figures are example embodiments wherein the like elements are numbered alike.

Features and aspects of embodiments are described below with reference to the accompanying drawings, in which elements are not necessarily depicted to scale.

Example embodiments of the present disclosure are further described with reference to the appended figures. To assist those of ordinary skill in the art in making and using the disclosed systems, assemblies and methods, reference is made to the appended figures, wherein:
FIG. 1 is a schematic of an example air management system, according to the present disclosure; and
FIG. 2 is a partial schematic from an example air management system, according to the present disclosure.

### DETAILED DESCRIPTION

The example embodiments disclosed herein are illustrative of air management systems, and assemblies of the present disclosure and methods/techniques thereof. It should be understood, however, that the disclosed embodiments are merely examples of the present disclosure, which may be embodied in various forms. Therefore, details disclosed herein with reference to example air management systems and associated processes/techniques of fabrication/assembly and use are not to be interpreted as limiting, but merely as the basis for teaching one skilled in the art how to make and use the systems/assemblies and/or alternative systems/assemblies of the present disclosure.

The present disclosure provides for air management systems, assemblies and methods for aircraft, with the air management systems/assemblies having negative ion technology for improved air quality.

In general, current practice provides that some available air conditioning/management systems in aircraft cannot handle fume events (e.g., abnormal odors, smoke, haze or fumes in the cabin) that may arise from various internal or external sources, and some may be due to contamination of the bleed air supply (e.g., as a result of a failure of an oil seal in the engine). Moreover, some available air conditioning/management systems in aircraft cannot handle harmful viruses/bacteria efficiently.

The present disclosure provides improved air conditioning/management systems having features to handle the poisonous/toxic chemicals/gases (e.g., carbon monoxide), climate relevant gases (e.g., methane), and/or harmful bacteria/viruses (e.g., SARS-CoV-2), and to improve the air quality inside the aircraft through the disclosed air conditioning/management systems.

As noted, some people experience acute symptoms due to a fume event or exposure to contaminants in conventional aircraft cabin air. Some of the chemical contaminants that are present during such events are irritants, and may cause itching or soreness of the eyes, nasal discharge, sore throat or coughing.

For example, turbine engine oil is an irritant and can include neurotoxic chemicals (e.g., tricresyl phosphate). Moreover, hydraulic fluid, although typically non-toxic in small quantities, can be extremely irritating to the eyes and skin, and can create a hazard to pilots during a fume event (but may cause no lasting damage). Furthermore, deicing fluid has a strong smell, but is not very irritating or toxic if inhaled (as opposed to the significant toxicity when ingested).

A U.S. Federal Aviation Administration (FAA) funded study found that fume events occur on 1 in 5,000 flights, and that some planes may have multiple consecutive events if the leak is not fixed. Airlines are required to file Service Difficulty Reports (SDRs) when smoke, vapor or noxious odors enter the cockpit or passenger cabin. In 2018, U.S. airlines conducted more than 12 million flights. The FAA's SDR database showed 232 submissions from air carriers that referenced a fume event that year.

The FAA requires manufacturers to show that the airplane crew and passenger compartment air is free from harmful or hazardous concentrations of gases and vapors.

As noted, currently available air conditioning/management systems in aircraft cannot handle the poisonous/toxic chemicals/gases and/or harmful viruses/bacteria efficiently. Currently used HEPA filters in cabin air conditioning and temperature control systems cannot trap/kill all of the viruses, and a significant percentage of the viruses will pass through these HEPA filters and circulate back into the cabin. In some cases, some of the trapped viruses on the HEPA filter will be blown through the HEPA filter into the cabin with the next wave of air. Due to the long-surviving time of viruses like SARS-CoV-2 in air, these particles linger for anywhere up to several hours, and this may cause the outbreak of harmful viruses/bacteria.

The present disclosure provides for air management systems, assemblies and methods (e.g., for aircraft or the like), with the air management systems/assemblies having negative ion technology for improved air quality.

The air management systems of the present disclosure have a negative ion generator that generates negative ions that mix with the cabin air. As such and after joint union/absorption of generated negative ions with positive ions (e.g., dust, microbes, airborne bacteria, allergens, viruses, etc.), the joint union/absorption of generated negative ions with positive ions become too heavy to float in the air and they will fall to the ground. With respect to smoke, odors, and/or poisonous/toxic chemicals, the joint union of these components with the generated negative ions will cause these components to become chemically diluted.

The air management systems of the present disclosure have a negative ion generator that generates negative hydroxyl radicals or ions (OH- ions).

The negative ion generator collects (invisible) moisture in the air (or water), and applies high voltage to it, thereby generating capsules of water including an abundance of (e.g., huge volumes of) negative OH- radicals/ions. Nano-sized capsules of water enveloping large amounts of negative OH- radicals/ions can be released all together into the air. The more negative OH- radicals/ions there are, then more bacteria/viruses and/or poisonous/toxic chemicals/gases in the cabin air can be effectively inhibited.

As the released nano-sized capsules of water (e.g., including large volumes of negative OH- radicals/ions) reach the pollutants in the air, the negative OH-radicals/ions react by transforming hydrogen from the pollutants into water, and this helps to inhibit growth of bacterial activity and/or reduces the concentrations of toxic gases/chemicals in the air.

It is noted that negative OH- radicals/ions have outstanding performance on odor removal and can inhibit activity of bacteria and viruses, and the negative OH-radicals/ions can control the concentrations of toxic gases (e.g., carbon monoxide and/or climate relevant gases such as methane or the like).

As discussed further below, the example air management/conditioning systems of the present disclosure can spread these nano-sized capsules of water (e.g., containing large volumes of negative OH- radicals/ions) by air flow of the internal air conditioners inside the aircraft or the like.

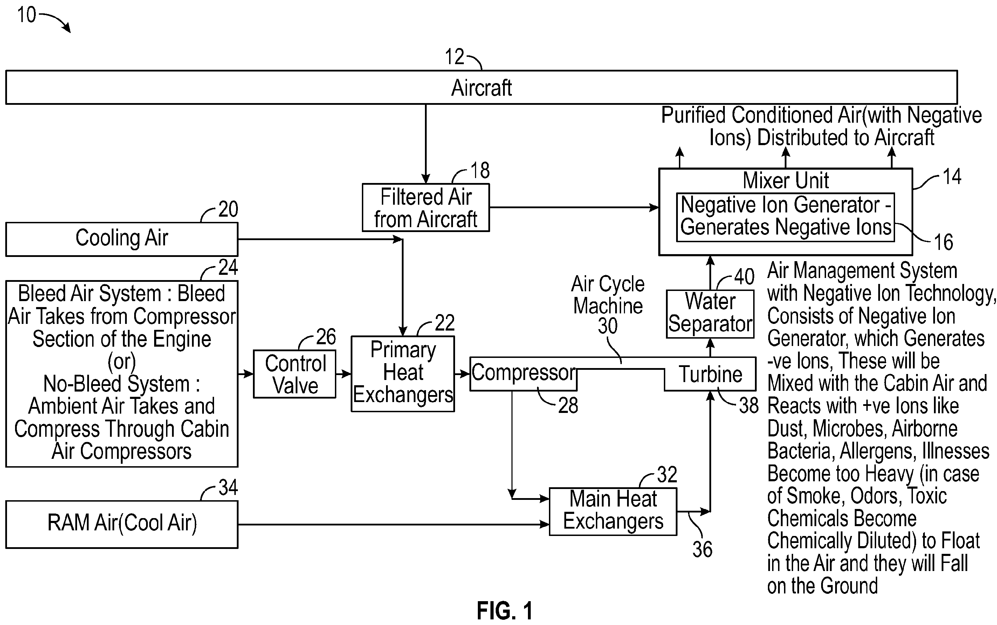

FIG. 1 is a schematic of an example air management system 10 of the present disclosure. In general, air management system 10 is an air management system 10 for aviation (e.g., for aircraft or the like), with the air management system 10 having negative ion technology for improved air quality, as discussed further below.

As shown in FIG. 1, the air management system 10 includes an aircraft cabin 12 in fluid communication with a mixer unit 14. The mixer unit 14 is in fluid communication with a negative ion generator 16, and the mixer unit 14 is in fluid communication with filtered air 18 from the aircraft cabin 12.

In example embodiments, the air management system 10 includes a stream of cooling air 20 in fluid communication with at least one primary heat exchanger 22, and an air feed stream 24 in fluid communication with the at least one primary heat exchanger 22 via a control valve 26.

It is noted that in some embodiments, the air feed stream 24 can be a bleed air feed stream 24 from a bleed air system, where bleed air is provided by a compressor section of the aircraft engine. In other embodiments, the air feed stream 24 can be an ambient air feed stream 24 provided by a no-bleed system where ambient air is compressed through one or more cabin air compressors.

As shown in FIG. 1, the air feed stream 24 and the cooling air stream 20 are fed to a compressor 28 of an air cycle machine 30. The compressor 28 then compresses the streams 20, 24, and feeds the compressed air to at least one main heat exchanger 32. A ram air stream 34 is also fed to the at least one main heat exchanger 32. After the at least one main heat exchanger 32, a cooled air stream 36 is fed to a turbine 38 of the air cycle machine 30, and then is fed to a water separator 40 and then to the mixer unit 14.

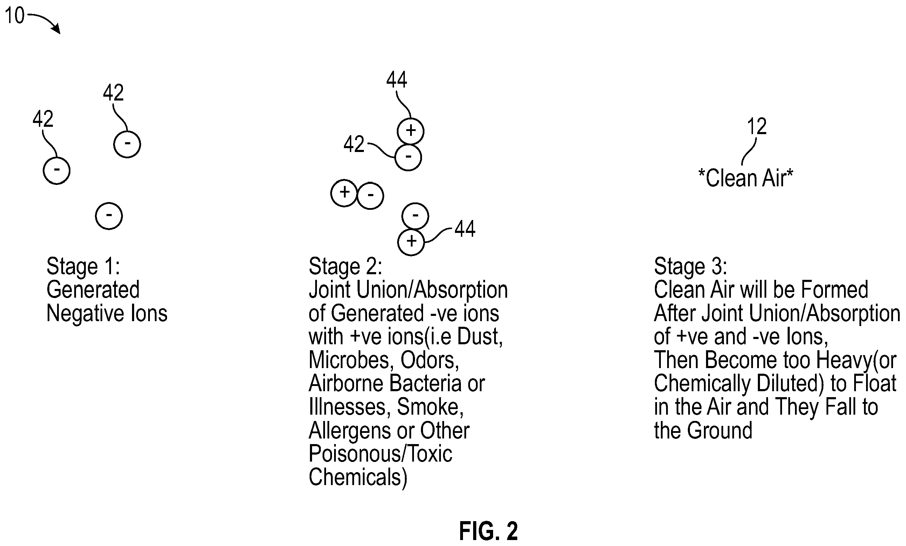

In general and as also shown in FIG. 2, the negative ion generator 16 generates negative ions 42 that mix with the filtered cabin air 18 in the mixer unit 14 and with the cooled air stream 36 fed to the mixer unit 14. FIG. 2 is a partial schematic from an example air management system 10, according to the present disclosure.

As such and after joint union/absorption of generated negative ions 42 with positive ions 44 (e.g., dust, microbes, airborne bacteria, allergens, viruses, etc.) from the filtered cabin air 18 and from air in the cabin 12, the joint union/absorption of generated negative ions 42 with positive ions 44 become too heavy to float in the air in the cabin 12 and they will fall to the ground. With respect to smoke, odors, and/or poisonous/toxic chemicals, the joint union of these components 44 with the generated negative ions 42 will cause these components to become chemically diluted in the air in the cabin 12.

The negative ion generator 16 generates negative hydroxyl radicals or ions (OH- ions) 42. The negative ion generator 16 collects (invisible) moisture in the air 18, 36 (or water), and applies high voltage to it, thereby generating capsules of water including an abundance of (e.g., large volumes of) negative OH- radicals/ions 42. Nano-sized capsules of water enveloping large amounts of negative OH- radicals/ions 42 can be released all together into the air via the mixer unit 14 and sent to the cabin 12. The more negative OH-radicals/ions 42 there are, then more bacteria/viruses and/or poisonous/toxic chemicals/gases in the air in the cabin 12 can be effectively inhibited.

As the released nano-sized capsules of water (e.g., including large volumes of negative OH- radicals/ions 42) reach the pollutants in the air, the negative OH- radicals/ions 42 react by transforming hydrogen from the pollutants into water, and this helps to inhibit growth of bacterial/virus activity and/or reduces the concentrations of toxic gases/chemicals in the air in the cabin 12. The negative OH-radicals/ions 42 have outstanding performance on odor removal and can inhibit activity of bacteria and viruses, and the negative OH- radicals/ions 42 can control the concentrations of toxic gases (e.g., carbon monoxide and/or climate relevant gases such as methane or the like) in the air of cabin 12.

As such and as shown in FIG. 1, the example air management system 10 can spread these nano-sized capsules of water (e.g., containing large volumes of negative OH- radicals/ions 42) by air flow of the internal air conditioners inside the aircraft of system 10.

There are many benefits of the systems, assemblies and methods of the present disclosure, including, without limitation, improving the air quality inside the aircraft through the air management systems by controlling the concentration of poisonous/toxic chemicals/gases (e.g., carbon monoxide and/or climate relevant gases like methane or the like); the air management systems facilitate with odor removal and/or inhibit activity of bacteria and/or viruses; the air management systems can avoid the outbreak of harmful viruses like SARS-CoV-2 (which has long-surviving time in air); and/or the air management systems can avoid or reduce the effects of fume events.

The ranges disclosed herein are inclusive of the endpoints, and the endpoints are independently combinable with each other (e.g., ranges of "up to 25 wt.%, or, more specifically, 5 wt.% to 20 wt.%", is inclusive of the endpoints and all intermediate values of the ranges of "5 wt.% to 25 wt.%," etc.). "Combinations" is inclusive of blends, mixtures, alloys, reaction products, and the like. The terms "first," "second," and the like, do not denote any order, quantity, or importance, but rather are used to distinguish one element from another. The terms "a" and "an" and "the" do not denote a limitation of quantity and are to be construed to cover both the singular and the plural, unless otherwise indicated herein or clearly contradicted by context. "Or" means "and/or" unless clearly stated otherwise. Reference throughout the specification to "some embodiments", "an embodiment", and so forth, means that a particular element described in connection with the embodiment is included in at least one embodiment described herein, and may or may not be present in other embodiments.

Unless defined otherwise, technical and scientific terms used herein have the same meaning as is commonly understood by one of skill in the art to which this application belongs.

Although the systems and methods of the present disclosure have been described with reference to example embodiments thereof, the present disclosure is not limited to such example embodiments and/or implementations. Rather, the assemblies, systems and methods of the present disclosure are susceptible to many implementations and applications within the scope of the invention as defined by the appended claims.

## Claims

1. An air management system comprising:
a mixer unit (14) in fluid communication with an aircraft cabin (12), and the mixer unit (14) in fluid communication with a negative ion generator (16) and with filtered air from the aircraft cabin (12);
wherein the negative ion generator (16) is configured to generate negative ions that mix with the filtered air from the aircraft cabin (12) in the mixer unit (14), and the mixer unit (14) is configured to send the mixed air to the aircraft cabin (12); and
wherein after joint union or absorption of the generated negative ions with positive ions from the mixed air, the joint union or absorption of the generated negative ions with the positive ions inhibits growth of bacterial or virus activity and reduces concentrations of toxic gases or chemicals in the mixed air in the aircraft cabin (12), and **characterized in that** the negative ion generator (16) is configured to generate negative hydroxyl radicals or ions and nano-sized capsules of water enveloping amounts of the negative hydroxyl radicals or ions.

2. The system of claim 1, wherein the joint union or absorption of the generated negative ions with the positive ions is configured to inhibit growth of SARS-CoV-2 in the mixed air in the aircraft cabin (12).

3. The system of any preceding claim, wherein the joint union or absorption of the generated negative ions with the positive ions is configured to reduce concentrations of at least one of smoke, vapor, haze, fumes, dust, allergens, abnormal odors, carbon monoxide, methane or tricresyl phosphate in the mixed air in the aircraft cabin (12).

4. The system of any preceding claim, wherein the joint union or absorption of the generated negative ions with the positive ions is configured to inhibit growth of microbes or airborne bacteria.

5. The system of any preceding claim, wherein the negative hydroxyl radicals or ions react by transforming hydrogen from components in the mixed air into water.

6. The system of any preceding claim, wherein the mixer unit (14) is in fluid communication with a cooled air stream from a turbine of an air cycle machine.

7. The system of claim 6, wherein the air cycle machine comprises a compressor, and the compressor is in fluid communication with an air feed stream; and
wherein the air feed stream comprises a bleed air feed stream from a bleed air system or comprises an ambient air feed stream from a no-bleed system.

8. The system of claim 6, wherein the turbine is in fluid communication with a ram air stream that is fed to the turbine via at least one main heat exchanger.

9. A method for operating an air management system comprising:
providing a mixer unit (14) in fluid communication with an aircraft cabin (12), and the mixer unit (14) in fluid communication with a negative ion generator (16) and with filtered air from the aircraft cabin (12);
generating negative ions via the negative ion generator (16);
mixing the generated negative ions with the filtered air from the aircraft cabin (12) in the mixer unit (14);
sending the mixed air to the aircraft cabin (12) via the mixer unit (14); and
wherein after joint union or absorption of the generated negative ions with positive ions from the mixed air, the joint union or absorption of the generated negative ions with the positive ions inhibits growth of bacterial or virus activity and reduces concentrations of toxic gases or chemicals in the mixed air in the aircraft cabin (12), and **characterized in that** the negative ion generator (16) generates negative hydroxyl radicals or ions and nano-sized capsules of water enveloping amounts of the negative hydroxyl radicals or ions.

10. The method of claim 9, wherein the joint union or absorption of the generated negative ions with the positive ions inhibits growth of SARS-CoV-2 in the mixed air in the aircraft cabin (12).

11. The method of any of claims 9 or 10, wherein the joint union or absorption of the generated negative ions with the positive ions reduces concentrations of at least one of smoke, vapor, haze, fumes, dust, allergens, abnormal odors, carbon monoxide, methane or tricresyl phosphate in the mixed air in the aircraft cabin (12).

12. The method of any of claims 9 to 11, wherein the joint union or absorption of the generated negative ions with the positive ions inhibits growth of microbes or airborne bacteria.

13. The method of any of claims 9 to 12, wherein the negative hydroxyl radicals or ions react by transforming hydrogen from components in the mixed air into water.

14. The method of any of claims 9 to 13, wherein the mixer unit (14) is in fluid communication with a cooled air stream from a turbine of an air cycle machine, and wherein the air cycle machine comprises a compressor, and the compressor is in fluid communication with an air feed stream; and
wherein the air feed stream comprises a bleed air feed stream from a bleed air system or comprises an ambient air feed stream from a no-bleed system, or wherein the turbine is in fluid communication with a ram air stream that is fed to the turbine via at least one main heat exchanger.

## Patentansprüche

1. Luftmanagementsystem, umfassend:
eine Mischereinheit (14) in Fluidverbindung mit einer Luftfahrzeugkabine (12), und die Mischereinheit (14) in Fluidverbindung mit einem Negativionengenerator (16) und mit gefilterter Luft aus der Luftfahrzeugkabine (12);
wobei der Negativionengenerator (16) dazu ausgelegt ist, negative Ionen zu erzeugen, die sich mit der gefilterten Luft aus der Luftfahrzeugkabine (12) in der Mischereinheit (14) mischen, und die Mischereinheit (14) dazu ausgelegt ist, die gemischte Luft an die Luftfahrzeugkabine (12) zu senden; und
wobei nach gemeinsamer Verbindung oder Absorption der erzeugten negativen Ionen mit positiven Ionen aus der gemischten Luft die gemeinsame Verbindung oder Absorption der erzeugten negativen Ionen mit den positiven Ionen Wachstum von bakterieller oder Virusaktivität hemmt und Konzentrationen von toxischen Gasen oder Chemikalien in der gemischten Luft in der Luftfahrzeugkabine (12) reduziert, und **dadurch gekennzeichnet, dass** der Negativionengenerator (16) dazu ausgelegt ist, negative Hydroxylradikale oder Ionen und nanoskalige Kapseln aus Wasser, die Mengen der negativen Hydroxylradikale oder Ionen umhüllend, zu erzeugen.

2. System nach Anspruch 1, wobei die gemeinsame Verbindung oder Absorption der erzeugten negativen Ionen mit den positiven Ionen dazu ausgelegt ist, Wachstum von SARS-CoV-2 in der gemischten Luft in der Luftfahrzeugkabine (12) zu hemmen.

3. System nach einem der vorhergehenden Ansprüche, wobei die gemeinsame Verbindung oder Absorption der erzeugten negativen Ionen mit den positiven Ionen dazu ausgelegt ist, Konzentrationen von mindestens einem von Rauch, Dampf, Dunst, Schwaden, Staub, Allergenen, abnormalen Gerüchen, Kohlenmonoxid, Methan oder Trikresylphosphat in der gemischten Luft in der Luftfahrzeugkabine (12) zu reduzieren.

4. System nach einem der vorhergehenden Ansprüche, wobei die gemeinsame Verbindung oder Absorption der erzeugten negativen Ionen mit den positiven Ionen dazu ausgelegt ist, Wachstum von Mikroben oder luftgetragenen Bakterien zu hemmen.

5. System nach einem der vorhergehenden Ansprüche, wobei die negativen Hydroxylradikale oder Ionen reagieren, indem sie Wasserstoff aus Bestandteilen in der gemischten Luft in Wasser umwandeln.

6. System nach einem der vorhergehenden Ansprüche, wobei die Mischereinheit (14) in Fluidverbindung mit einem gekühlten Luftstrom von einer Turbine einer Luftkreislaufmaschine ist.

7. System nach Anspruch 6, wobei die Luftkreislaufmaschine einen Verdichter umfasst und der Verdichter in Fluidverbindung mit einem Luftzufuhrstrom ist; und
wobei der Luftzufuhrstrom einen Zapfluftzufuhrstrom von einem Zapfluftsystem umfasst oder einen Umgebungsluftzufuhrstrom von einem zapfluftfreien System umfasst.

8. System nach Anspruch 6, wobei die Turbine in Fluidverbindung mit einem Stauluftstrom ist, der der Turbine über mindestens einen Hauptwärmetauscher zugeführt wird.

9. Verfahren zum Betreiben eines Luftmanagementsystems, umfassend:
Bereitstellen einer Mischereinheit (14) in Fluidverbindung mit einer Luftfahrzeugkabine (12), und der Mischereinheit (14) in Fluidverbindung mit einem Negativionengenerator (16) und mit gefilterter Luft aus der Luftfahrzeugkabine (12);
Erzeugen negativer Ionen über den Negativionengenerator (16);
Mischen der erzeugten negativen Ionen mit der gefilterten Luft aus der Luftfahrzeugkabine (12) in der Mischereinheit (14);
Senden der gemischten Luft an die Luftfahrzeugkabine (12) über die Mischereinheit (14); und
wobei nach gemeinsamer Verbindung oder Absorption der erzeugten negativen Ionen mit positiven Ionen aus der gemischten Luft die gemeinsame Verbindung oder Absorption der erzeugten negativen Ionen mit den positiven Ionen Wachstum von bakterieller oder Virusaktivität hemmt und Konzentrationen von toxischen Gasen oder Chemikalien in der gemischten Luft in der Luftfahrzeugkabine (12) reduziert, und **dadurch gekennzeichnet, dass** der Negativionengenerator (16) negative Hydroxylradikale oder Ionen und nanoskalige Kapseln aus Wasser, die Mengen der negativen Hydroxylradikale oder Ionen umhüllend, erzeugt.

10. Verfahren nach Anspruch 9, wobei die gemeinsame Verbindung oder Absorption der erzeugten negativen Ionen mit den positiven Ionen Wachstum von SARS-CoV-2 in der gemischten Luft in der Luftfahrzeugkabine (12) hemmt.

11. Verfahren nach einem der Ansprüche 9 oder 10, wobei die gemeinsame Verbindung oder Absorption der erzeugten negativen Ionen mit den positiven Ionen Konzentrationen von mindestens einem von Rauch, Dampf, Dunst, Schwaden, Staub, Allergenen, abnormalen Gerüchen, Kohlenmonoxid, Methan oder Trikresylphosphat in der gemischten Luft in der Luftfahrzeugkabine (12) reduziert.

12. Verfahren nach einem der Ansprüche 9 bis 11, wobei die gemeinsame Verbindung oder Absorption der erzeugten negativen Ionen mit den positiven Ionen Wachstum von Mikroben oder luftgetragenen Bakterien hemmt.

13. Verfahren nach einem der Ansprüche 9 bis 12, wobei die negativen Hydroxylradikale oder Ionen reagieren, indem sie Wasserstoff aus Bestandteilen in der gemischten Luft in Wasser umwandeln.

14. Verfahren nach einem der Ansprüche 9 bis 13, wobei die Mischereinheit (14) in Fluidverbindung mit einem gekühlten Luftstrom von einer Turbine einer Luftkreislaufmaschine ist, und wobei die Luftkreislaufmaschine einen Verdichter umfasst und der Verdichter in Fluidverbindung mit einem Luftzufuhrstrom ist; und wobei der Luftzufuhrstrom einen Zapfluftzufuhrstrom von einem Zapfluftsystem umfasst oder einen Umgebungsluftzufuhrstrom von einem zapfluftfreien System umfasst, oder wobei die Turbine in Fluidverbindung mit einem Stauluftstrom ist, der der Turbine über mindestens einen Hauptwärmetauscher zugeführt wird.

## Revendications

1. Système de gestion de l'air comprenant :
une unité de mélangeur (14) en communication fluidique avec une cabine d'aéronef (12), et l'unité de mélangeur (14) en communication fluidique avec un générateur d'ions négatifs (16) et avec l'air filtré provenant de la cabine d'aéronef (12) ;
dans lequel le générateur d'ions négatifs (16) est configuré pour générer des ions négatifs qui se mélangent à l'air filtré provenant de la cabine d'aéronef (12) dans l'unité de mélangeur (14), et l'unité de mélangeur (14) est configurée pour envoyer l'air mélangé à la cabine d'aéronef (12) ; et
dans lequel après l'union ou absorption conjointe des ions négatifs générés avec des ions positifs provenant de l'air mélangé, l'union ou absorption conjointe des ions négatifs générés avec les ions positifs inhibe une croissance d'activité bactérienne ou virale et réduit des concentrations de gaz ou de produits chimiques toxiques dans l'air mélangé de la cabine d'aéronef (12), et **caractérisé en ce que** le générateur d'ions négatifs (16) est configuré pour générer des radicaux ou ions hydroxyles négatifs et des capsules de taille nanométrique d'eau enveloppant des quantités des radicaux ou ions hydroxyles négatifs.

2. Système selon la revendication 1, dans lequel l'union ou absorption conjointe des ions négatifs générés avec les ions positifs est configurée pour inhiber la croissance du SARS-CoV-2 dans l'air mélangé de la cabine d'aéronef (12).

3. Système selon une quelconque revendication précédente, dans lequel l'union ou absorption conjointe des ions négatifs générés avec les ions positifs est configurée pour réduire des concentrations d'au moins l'un de fumée, vapeur, brume, émanation, poussière, allergènes, odeurs anormales, monoxyde de carbone, méthane ou phosphate de tricrésyle dans l'air mélangé de la cabine d'aéronef (12).

4. Système selon une quelconque revendication précédente, dans lequel l'union ou absorption conjointe des ions négatifs générés avec les ions positifs est configurée pour inhiber une croissance de microbes ou de bactéries aéroportées.

5. Système selon une quelconque revendication précédente, dans lequel les radicaux ou ions hydroxyles négatifs réagissent en transformant l'hydrogène provenant de composants de l'air mélangé en eau.

6. Système selon une quelconque revendication précédente, dans lequel l'unité de mélangeur (14) est en communication fluidique avec un flux d'air refroidi provenant d'une turbine d'une machine à cycle d'air.

7. Système selon la revendication 6, dans lequel la machine à cycle d'air comprend un compresseur, et le compresseur est en communication fluidique avec un flux d'alimentation en air ; et dans lequel le flux d'alimentation en air comprend un flux d'alimentation en air de prélèvement provenant d'un système d'air de prélèvement ou comprend un flux d'alimentation en air ambiant provenant d'un système sans prélèvement.

8. Système selon la revendication 6, dans lequel la turbine est en communication fluidique avec un flux d'air dynamique qui est alimenté à la turbine par l'intermédiaire d'au moins un échangeur de chaleur principal.

9. Procédé de fonctionnement d'un système de gestion de l'air comprenant :
la fourniture d'une unité de mélangeur (14) en communication fluidique avec une cabine d'aéronef (12), et l'unité de mélangeur (14) en communication fluidique avec un générateur d'ions négatifs (16) et avec l'air filtré de la cabine d'aéronef (12) ;
la génération d'ions négatifs par l'intermédiaire du générateur d'ions négatifs (16) ;
le mélange des ions négatifs générés avec l'air filtré provenant de la cabine d'aéronef (12) dans l'unité de mélangeur (14) ;
l'envoi de l'air mélangé à la cabine d'aéronef (12) par l'intermédiaire de l'unité de mélangeur (14) ; et
dans lequel après l'union ou absorption conjointe des ions négatifs générés avec des ions positifs provenant de l'air mélangé, l'union ou absorption conjointe des ions négatifs générés avec les ions positifs inhibe une croissance d'activité bactérienne ou virale et réduit des concentrations de gaz ou de produits chimiques toxiques dans l'air mélangé de la cabine d'aéronef (12), et **caractérisé en ce que** le générateur d'ions négatifs (16) génère des radicaux ou ions hydroxyles négatifs et des capsules de taille nanométrique d'eau enveloppant des quantités des radicaux ou ions hydroxyles négatifs.

10. Procédé selon la revendication 9, dans lequel l'union ou absorption conjointe des ions négatifs générés avec les ions positifs inhibe la croissance du SARS-CoV-2 dans l'air mélangé de la cabine d'aéronef (12).

11. Procédé selon l'une quelconque des revendications 9 ou 10, dans lequel l'union ou absorption conjointe des ions négatifs générés avec les ions positifs réduit des concentrations d'au moins l'un de fumée, vapeur, brume, émanation, poussière, allergènes, odeurs anormales, monoxyde de carbone, méthane ou phosphate de tricrésyle dans l'air mélangé de la cabine d'aéronef (12).

12. Procédé selon l'une quelconque des revendications 9 à 11, dans lequel l'union ou absorption conjointe des ions négatifs générés avec les ions positifs inhibe la croissance de microbes ou de bactéries aéroportées.

13. Procédé selon l'une quelconque des revendications 9 à 12, dans lequel les radicaux ou ions hydroxyles négatifs réagissent en transformant l'hydrogène provenant de composants de l'air mélangé en eau.

14. Procédé selon l'une quelconque des revendications 9 à 13, dans lequel l'unité de mélangeur (14) est en communication fluidique avec un flux d'air refroidi provenant d'une turbine d'une machine à cycle d'air, et dans lequel la machine à cycle d'air comprend un compresseur, et le compresseur est en communication fluidique avec un flux d'alimentation en air ; et dans lequel le flux d'alimentation en air comprend un flux d'alimentation en air de prélèvement provenant d'un système d'air de prélèvement ou comprend un flux d'alimentation en air ambiant provenant d'un système sans prélèvement, ou dans lequel la turbine est en communication fluidique avec un flux d'air dynamique qui est alimenté à la turbine par l'intermédiaire d'au moins un échangeur de chaleur principal.
